# EUROPEAN PATENT APPLICATION

(11) **EP 3 415 156 A1**
(43) Date of publication of application: **19.12.2018**
(21) Application number: 18177974.5
(22) Date of filing: 15.06.2018
(51) Int. Cl.: A61K 36/752, A61P 1/16

(54) **NOVEL EXTRACTS OF BERGAMOT ORANGE, COMBINATIONS THEREOF AND FORMULATIONS CONTAINING THEM**

(30) Priority: 16.06.2017 IT 201700067461
(71) Applicant: Herbal E Antioxidant Derivatives S.R.L. Ed in Form Abbreviata H&AD S.R.L., 89032 Bianco (RC) (IT)
(72) Inventor: MOLLACE, Vincenzo, 89032 BIANCO (RC) (IT)
(74) Representative: Bianchetti Bracco Minoja S.r.l.

(57) **Abstract**

Disclosed is an extract of *Citrus aurantium* var. *bergamia* which is useful for the prevention and treatment of dyslipidaemia, hyperglycaemia and vascular inflammation, and especially of hepatic steatosis. The extract according to the invention is distinguished by a high content of melitidin and brutieridin and oligomeric polyphenols. The extract is obtained from the biomass after prior freezing and thawing of the squeezed fruit.

## Description

### Field of invention

The present invention relates to an extract of *Citrus aurantium* var. *bergamia* which is useful for the prevention and treatment of dyslipidaemia, hyperglycaemia and vascular inflammation, and especially of hepatic steatosis.

### Prior art

Extracts of *Citrus aurantium* var. *bergamia,* particularly those described in US 8,741,362, contain a complex polyphenol mixture whose main ingredients, neoeriocitrin, naringin and neohesperidin, constitute 40% to 50% by weight of the extract. A further fraction amounting to about 10% of the total extract comprises other flavonoids, some of which (melitidin and brutieridin) are characterised by a 3-hydroxy-3-methylglutaryl residue bonded to a hydroxy group of the glycoside portion. The cholesterol synthesis-inhibiting properties demonstrated for bergamot orange extracts are attributed to said structural characteristic.

In particular, the plant compound prepared according to US 8,741,362 from the juice of fresh bergamot oranges has proved useful not only to normalise the lipid and blood glucose parameters in patients suffering from diet-dependent or familial genetic metabolic disorders, but also for the treatment of vascular and hepatic inflammatory processes (Mollace et al., Fitoterapia, vol. 82, no. 3, 2011, pp. 309-316).

In pathological hyperlipaemia, a reduction in total cholesterol and LDL cholesterol has been demonstrated at concentrations ranging between 20 and 32%, with a 30% increase in HDL cholesterol. In addition to these important variations a marked reduction in VLDL cholesterol and a dimensional increase in LDLs has been found, leading to a reduction in their oxidation.

The process described in US 8,741,362 leads to almost complete elimination of furanocoumarins and coumarins, which are potentially responsible for allergic reactions or adverse effects on coagulation and the blood count.

According to said process, the fruit, after removal of the external glandular part containing the essential oil, is coarsely crushed and pressed. The resulting juice then undergoes depectinising enzymatic treatment. A liquid chromatographable on absorption columns is obtained after serial centrifugation. Re-elution with alkalis gives rise to the final extract.

WO 2015/136441 discloses an extract of a variety of bergamot orange (Risso & Poiteau) having a high content of flavonoids, in particular brutieridin and melitidin. The presence of oligomeric polyphenols has not been reported.

Although the extracts described to date are active in controlling hyperlipidaemia, they have not proved promising in the treatment of metabolic syndrome or hepatic steatosis.

### Description of the invention

It has now been discovered that an extract of bergamot orange enriched in a fraction of oligomeric polyphenols having molecular weights ranging between 1100 and 2500 is particularly effective in the treatment of metabolic syndrome and hepatic steatosis.

The subject of the invention is therefore an extract of *Citrus aurantium* var. *bergamia* characterised by:
- a total HPLC-assayable flavonoid content ranging between 35 and 65%;
- a total flavonoid content, expressed as neoeriocitrin, of 75%, determined by the Folin-Ciocalteu method;
- a neoeriocitrin, naringin, neohesperidin, melitidin and brutieridin content of not less than 40%, melitidin and brutieridin constituting 15 to 25% by weight of the total mixture of neoeriocitrin, naringin, neohesperidin, melitidin and brutieridin;
- a content of oligomeric polyphenols with molecular weights of 1100 to 2500 ranging between 25 and 50% by weight.

The flavonoids are assayed by the HPLC method on a Kinetex 5µ C18 100A (150 x 4.6) mm column with a gradient of acetonitrile (eluent A) and an 0.088% solution of acetic acid in water (v:v) (eluent B), injection volume 3.0 µL (methanol:water 1:1), temperature 30° ± 1°C.

The elution gradient is shown in the table.

| **Step** | **time (min)** | **Flow (mL/min)** | **Eluent A (%)** | **Eluent B (%)** |
|---|---|---|---|---|
| **0** Equilibration | 6.0 | 1.4 | 7.0 | 93.0 |
| **1** Run | 2.0 | 1.4 | 19.0 | 81.0 |
| **2** Run | 8.0 | 1.4 | 20.4 | 79.6 |
| **3** Run | 3.0 | 1.4 | 60.0 | 40.0 |
| **4** Run | 1.0 | 1.4 | 100.0 | 0.0 |
| **5** Run | 3.0 | 1.4 | 100.0 | 0.0 |
| **6** Run | 1.0 | 1.4 | 7.0 | 93.0 |
| **6** Washing | 4.0 | 1.4 | 7.0 | 93.0 |

Under the experimental conditions employed, using a PerkinElmer Flexar Module system with photodiode detector and Chromera (r) analysis software, the retention times in minutes were 6.19 for neoeriocitrin, 7.81 for naringin, 9.02 for neohesperidin, 11.75 for melitidin and 13.21 for brutieridin.

The total flavonoids were determined by the Folin Ciocalteu colorimetric method (Int. J. Food Sci. Technol., 37 (2002), pp. 153-161; Evid Based Complement Alternat Med. 2015; 2015: 957031). Briefly, 50 *µ*L of methanol/water solutions of the samples was added to 450 *µ*L of deionised water, 500 *µ*L of Folin-Ciocalteu reagent (aqueous solution of phosphomolybdate and phosphotungstate) and 500 *µ*L of 10% sodium carbonate solution. After incubation in the dark for 1 h at room temperature, the absorbance is read off at 786 nm against a blank containing 50 *µ*L of the same solvent. The total phenol content is expressed in mg of gallic acid equivalents (GAE/g of dried extract).

The extract according to the invention can be obtained by applying substantially the same process as described in US 8,741,362, but modified by adding a heat-shock pretreatment involving freezing/thawing the biomass deriving from squeezing of the fruit (pulp).

The *Citrus aurantium* var. *bergamia* fruit, after removal of the outer glandular part containing the essential oil, is pressed to obtain the juice, while the solid residue (pulp) is rapidly frozen and then subjected to heat shock by enzymatic oxidase inhibition and release of hydrolase, and by increasing cell permeability. The biomass is pressed, and the aqueous phase containing the active and functional ingredients is recovered. The resulting aqueous extract is purified by reverse dialysis to remove undesirable substances that reduce the performance of the resins during the subsequent stages of the process.

In particular, the pulp remaining after squeezing of the fruit is rapidly frozen to -20°C and stored. The frozen biomass is rapidly thawed and heated to a temperature ranging between 15 and 60°C, preferably 30°C, and left to stand for a time ranging between 2 and 12 h, preferably 6 h, after which the biomass is pressed in a suitable machine at pressures ranging from 100 to 300 bars, preferably 200 bars. After pressing and counterwashes with water in countercurrent and depectinising enzyme treatment, the aqueous extract is centrifuged and filtered, and the clear filtrate is mainly treated with SEPABEADS SP absorption resin or other polystyrene resins; the resin, which retains the polyphenol substances, is washed thoroughly with water to remove inert substances and the retentate is then eluted with an aqueous solution of KOH which is immediately neutralised to a weakly acid pH at the outlet of the column. Said solution is atomised after elimination of the water in a vacuum.

Another subject of the invention is therefore a process for the preparation of a bergamot orange extract comprising:
a) mincing the bergamot orange fruit after removal of the outer cuticle and flavedo;
b) freezing the biomass (pulp) obtained in step a) at a temperature ranging between -10°C and -30 °C, preferably -20 ± 2°C;
c) thawing the biomass at a temperature ranging between 15 and 60°C, preferably 30°C, and maintaining the thawed biomass at the same temperature for a time ranging between 2 and 12h;
d) pressing the thawed biomass obtained in step c) at pressures ranging from 100 to 300 bars, preferably 200 bars;
e) inoculating pectin-degrading enzymes into the pressed biomass;
f) reducing the pulp content of the biomass obtained in step e) to under 0.5%;
g) deactivating the enzymes introduced in step e);
h) ultrafiltration of the biomass obtained in step g) with 30,000 dalton molecular weight cut-off membranes to give a clear solution;
i) passing the clear solution obtained in step e) through columns containing adsorbent polystyrene resins for adsorption of the polyphenols;
k) washing said columns with water at a temperature ranging between 30° and 50°C, and increasing the pH to values ranging between 12 and 14 using alkali metal hydroxides, to give an eluate;
l) passing said eluate through cation-exchange resins with consequent adjustment of pH to values below 3.0 and recovery of said eluate;
m) drying of eluate.

The resulting product has an HPLC-assayable flavonoid content ranging between 35 and 65%, normally 40%, and an assay value in total flavonoids, determined by the Folin-Ciocaulteu method, of 75% expressed as neoeriocitrin.

It is postulated that thawing of the pulp at room temperature gives rise to a different flavonoid distribution due to the rupture of the cell membranes. In particular, while the quantity of neoeriocitrin and naringin remains almost equal, a significant increase in melitidin, and an even more marked increase in neohesperidin and brutieridin is observed, compared with the extract obtained without the freezing/thawing steps.

However, the usefulness of the invention is in no way dependent on verification of said hypothetical action mechanism.

The final extract according to the invention exhibited a surprising activity against hepatic steatosis.

The pharmacological properties of the novel extract were compared with those of the extract described in US 8,741,362.

Normal rats, Zucker fatty rats and Zucker diabetic fatty (ZDF) rats were treated for that purpose.

The treatment was given for 30 days, and metabolic state, insulin sensitivity, glucose tolerability and hepatic steatosis were evaluated. Both extracts improved the parameters examined, but with a different intensity compared with the controls. During euglycaemic hyperinsulin treatment with the extract according to the invention (BE 75), the Zucker fatty rats and Zucker diabetic fatty rats required a higher infusion of glucose than the controls to maintain a stable blood glucose level (1.6 ± 0.23, 1.5 ± 0.18, 2.01 ± 0.19 and 4.1, %.1 and 6.42 ± 1.03 mg min⁻¹ respectively) compared with the control values (0.71 ± 0.17 mg min⁻¹ and 2.09 ± 0.71 p<0.05), demonstrating increased insulin sensitivity in both the Zucker fatty rats and the Zucker diabetic fatty rats.

The extract according to the invention exercises a surprising effect on non-alcoholic hepatic steatosis, while maintaining its activities on the lipid and glycometabolic parameters.

A group of 186 patients was also selected for treatment on the basis of inclusion criteria involving stable hyperlipaemia with an alcohol intake not exceeding 20 g a day and a fatty liver determined by abdominal ultrasound scan, including patients with a diffuse increase in liver echogenicity compared with kidney echogenicity and with a hepatorenal index between 2.5 and 3.5. One group was treated with the product according to the invention and one with the placebo. The patients selected had fasting LDL values exceeding 130 mg/dl, triglycerides exceeding 200 mg/dl and HDL lower than 40 mg/dl.

The clinical trial involved a treatment period of 16 weeks, with check-ups at time zero and at the end of treatment. The results are shown in tables 1-4.

**Table 1 - Basal characteristics of the subjects**

| **Parameters** | **Placebo (*n*=93)** | **Product of Example 1 (*n*=95)** |
|---|---|---|
| Age (years) | | |
| Mean | 52±10 | 51.0±9 |
| Weight (kg) | 78.8±0.7 | 79.2±0.8 |
| BMI (kg/m²) | 30±0.4 | 32.2±0.5 |
| Smokers (%) | 23 | 20 |
| Alcohol consumption (> 20 g/day) | 0 | 0 |

| | | |
|---|---|---|
| BMI, body mass index. | | |

**Table 2 - Effects of treatment on lipid profile**

| **Parameters** | **Placebo (*n*=93)** | **Product of Example 1 (*n*=95)** | ***P* Value** |
|---|---|---|---|
| TG (mg/dL) | | | |
| Baseline | 235±12 | 241±11 | |
| Δ16 weeks | -10±3 | -86±9 | <0.001 |
| TC (mg/dL) | | | |
| Baseline | 246±8 | 254±10 | |
| Δ16 weeks | -8±2 | -78±8 | <0.001 |
| LDL-C (mg/dL)C | | | |
| Baseline | 163±12 | 169±18 | |
| Δ16 weeks | -5±2 | -48±10 | <0.001 |
| HDL-C (mg/dL) | | | |
| Baseline | 38±3 | 38±4 | |
| Δ16 weeks | +3±0.4 | +13±0.7 | <0.001 |
| OxLDL (U/L) | | | |
| Baseline | 126±12 | 131±14 | |
| Δ16 weeks | -3±2 | -31±5 | <0.001 |
| TG, triglycerides; TC, total cholesterol; LDL-C, LDL cholesterol; HDL-C, HDL cholesterol; Ox LDL, oxidised LDL. | | | |

Data expressed as mean ± SD for each value; a P value of <0.05 after 16 weeks' treatment with the extract according to the invention compared with the placebo group was considered significant.

**Table 3 - Effects of treatments on liver damage parameters**

| **Parameter** | **Placebo** = **93)** | **Extract of Example 1 (*n*=95)** | **P Value** |
|---|---|---|---|
| ALP (U/L) | | | |
| Baseline | 65.26 + 4.5 | 66.61 + 5.2 | |
| Δ16 weeks | -1.29 ± 0.73 | -18 ± 1.85 | <0.001 |
| GGT (U/L) | | | |
| Baseline | 67.44 + 3.9 | 68.34 + 4.3 | |
| Δ16 weeks | -2.73 ± 1.03 | -18.76 ± 3.06 | <0.001 |
| ALT (U/L) | | | |
| Baseline | 53.51 + 4.1 | 54.87 + 4.7 | |
| Δ16 weeks | -0.31 ± 0.67 | -15.41 ± 2.68 | <0.001 |
| AST (U/L) | | | |
| Baseline | 42.73+ 3.9 | 44.58 + 4.2 | |
| 16 weeks | -0.43 ± 0.69 | -11.30 ± 0.71 | <0.001 |
| HA (ng/mL) | | | |
| Baseline | 85.69 + 10.1 | 82.63 + 9.6 | |
| Δ16 weeks | -2.78 ± 3.21 | 21.67 ± 4.29 | <0.001 |
| PC III (ng/mL) | | | |
| Baseline | 72.25 + 8.1 | 75.84 + 7.8 | |
| Δ16 weeks | -1.93 ± 1.45 | -18.77 ± 3.49 | <0.001 |
| IV-C (ng/mL) | | | |
| Baseline | 58.44 + 5.5 | 58.60 + 6.1 | |
| Δ16 weeks | -1.57 ± 1.26 | -16.23 ± 3.48 | <0.001 |
| Hepatorenal Index | | | |
| Baseline | 3.0 + 0.4 | 3.1 + 0.4 | |
| Δ16 weeks | -0.3 + 0.1 | -13.3 + 0.3 | <0.05 |

TB, total bilirubin; ALP, alkaline phosphatase; TP, total p-proteins; GGT, gamma-glutamyltransferase; ALT, alanine aminotransferase; AST, aspartate aminotransferase; HA, hyaluronic acid; PC-III, type III precollagen; IV-C, type IV collagen.

Data expressed as mean ± SD for each value; a P value of <0.05 after 16 weeks' treatment with the extract according to the invention compared with the placebo group was considered significant.

**Table 4 -Effects of treatments on GPx, SOD, MDA and TNF-α**

| **Parameters** | **Placebo (*n*=93)** | **Extract ES 1 (*n*** = **95)** | ***P* value** |
|---|---|---|---|
| GPx (U/mL) | | | |
| Baseline | 192.48 ± 3.88 | 188.15 ± 4.29 | |
| Δ9 weeks | 4.76 ± 2.63a | 25.97 ± 3.68 | <0.001 |
| SOD (U/mL) | | | |
| Baseline | 35.78 ± 4.1 | 33.33 ± 3.6 | |
| Δ9 weeks | 0.16 ± 0.72 | 9.83 ± 0.73 | <0.001 |
| MDA (nmol/mL) | | | |
| Baseline | 4.92 ± 0.4 | 4.84 ± 0.6 | |
| Δ9 weeks | -0.08 ± 0.05 | -1.41 ± 0.05 | <0.001 |
| TNF-α (pg/mL) | | | |
| Baseline | 92.85 + 8.5 | 98.10 + 9.2 | |
| Δ9 weeks | -4.65 ± 2.17 | -28.56 ± 2.22 | <0.001 |

GPx, glutathione peroxidase; SOD, superoxide dismutase; MDA, malondialdehyde; TNF, tumour necrosis factor alpha

Data expressed as mean ± SD for each value; a P value of <0.05 after 16 weeks' treatment with the extract according to the invention compared with the placebo group was considered significant.

The extract according to the invention can be combined with other standardised extracts and/or carotenoids. Examples of extracts which can be advantageously associated with bergamot orange extract include extracts of *Olea oleracea, Berberis aristata, Vitis vinifera, Cyclanthera pedata, Gymnema sylvestre* and *Eugenia jambolana.*

The formulations according to the invention have also proved effective on different parameters in a range of patients suffering from metabolic syndrome, in whom normalisation of parameters such as blood glucose, lipid parameters, hypertension and "silent inflammation" was observed.

According to a preferred aspect, the compositions according to the invention will be formulated as normal or gastroprotected capsules or tablets, to promote topical local activity while leaving the digestive function at stomach level unchanged.

These compounds can be administered to humans preferably in oils rich in ω-3 fatty acids and phospholipids to facilitate absorption of the polymer flavonoids of the *Citrus bergamia* extract.

According to a further aspect, the compositions according to the invention may be administered together with other substances having a useful or complementary activity.

The compositions according to the invention will be formulated according to conventional methods, such as those described in "Remington's Pharmaceutical Handbook", Mack Publishing Co., N.Y., USA. In particular, the compositions according to the invention will be formulated according to conventional plant ingredient formulation techniques, which require particular care to be taken to avoid interactions with the excipients and the capsule matrices. Examples of oral formulations are tablets, dragées, soft and hard gelatin capsules, and cellulose capsules.

The examples set out below further illustrate the invention.

### Example 1 - Preparation of Citrus aurantium var bergamia extract

100 kg of pulp is rapidly frozen to a temperature of -20 degrees. The frozen biomass is rapidly thawed to room temperature (25-30°C), and left to stand for 6 h. The biomass is pressed at 200 bars, separating the liquid and counterwashing the pressing with 2 volumes of demineralised water. The combined liquids are clarified by centrifugation and absorbed on SEPABEADS SP resin for concentration of the polyphenol substances; the resin is washed thoroughly with water to remove inert substances, and then eluted with 0.5N KOH; the basic solution is immediately neutralised with sulphone resin to pH 4.5 and concentrated in a vacuum until dry. The result is 0.75 Kg of a yellowish product having an assay value in total polyphenols of 75% expressed as neoeriocitrin with the Foling method, and the following content of the characteristic glycosylated flavanones neoeriocitrin, naringin, neohesperidin, melitidin and brutieridin:

| | |
|---|---|
| Neoeriocitrin | 25.2% |
| Naringin | 25.5% |
| Neohesperidin | 29.1% |
| Melitidin | 5.6% |
| Brutieridin | |
| 14.6% | |

### Example 2 - Preparation of 710 mg hard gelatin capsules

Unit composition:

| | |
|---|---|
| EXTRACT OF EXAMPLE 1 | 600 mg |
| Microcrystalline cellulose | 100 mg |
| Silicon dioxide | 5 mg |
| Magnesium stearate | 5 mg |

### Example 3 - 800 mg soft gelatin capsules

Unit composition

| | |
|---|---|
| Extract of example 1 | 300 mg |
| Soya lecithin | 200 mg |
| Linseed oil | 300 mg |

## Claims

1. An extract of *Citrus aurantium* var. *bergamia* **characterised by** having:
- a total flavonoid content assayable by HPLC ranging from 35 to 65%;
- a total flavonoid content expressed as neoeriocitrin, evaluated by the Folin-Ciocalteu method, of 75%;
- a neoeriocitrin, naringin, neohesperidin, melitidin and brutieridin content not lower than 40% of the total weight of the extract, melitidin and brutieridin constituting 15 to 25% by weight of the total neoeriocitrin, naringin, neohesperidin, melitidin and brutieridin mixture;
- a content of oligomeric polyphenols with molecular weights of 1100 to 2500 ranging between 25 and 50% by weight.

2. A process for the production of the extracts of claim 1 comprising:
a) mincing the bergamot orange fruit after removal of the outer cuticle and the flavedo;
b) freezing the biomass (*pulp*) obtained in step a) at a temperature ranging from - 10°C to -30 °C, preferably -20 ± 2 °C;
c) thawing the biomass at a temperature ranging from 15 to 60°C, preferably 30°C, and keeping the thawed biomass at the same temperature for a time ranging from 2 to 12 hrs;
d) pressing the thawed biomass obtained in step c) at pressures ranging from 100 to 300 bars, preferably 200 bars;
e) inoculating pectin-degrading enzymes into the pressed biomass;
f) reducing the pulp content of the biomass obtained in step e) below 0.5%;
g) deactivating the enzymes introduced in step e);
h) ultrafiltering the biomass obtained in step g) with 30,000 dalton molecular weight cut-off membranes to give a clear solution;
i) passing the clear solution obtained in step e) through columns containing adsorbent polystyrene resins to adsorb polyphenols;
k) washing said columns with water at temperatures ranging from 30° to 50°C and increasing the pH to values ranging from 12 to 14 by means of alkali metal hydroxides, to give an eluate;
l) passing said eluate through cationic resins, thereby adjusting the pH to values lower than 3.0, and recovering said eluate;
m) drying the eluate.

3. Extract obtained by the process of claim 2.

4. Compositions comprising the extracts of claim 1 or 3 in admixture with suitable carriers or excipients.

5. Compositions according to claim 4 further comprising carotenoids and/or standardised plant extracts.

6. Compositions according to claim 5 wherein the standardised extracts are selected from extracts of *Olea* spp., *Berberis aristata, Vitis vinifera, Cyclanthera pedata, Gymnema silvestre* and *Eugenia jambolana.*

7. Extracts of claim 1 or 3 for use in the treatment of hepatic steatosis.
